Europäisches Patentamt

European Patent Office

Office européen des brevets

(19)

(11) Veröffentlichungsnummer: **0 007 995**
A1

(12) # EUROPÄISCHE PATENTANMELDUNG

(21) Anmeldenummer: 79102213.0

(22) Anmeldetag: 02.07.79

(51) Int. Cl.³: **A 61 K 31/16**

(30) Priorität: 14.08.78 AT 5914/78

(43) Veröffentlichungstag der Anmeldung: 20.02.80
Patentblatt 80/4

(84) Benannte Vertragsstaaten: **AT BE CH DE FR GB IT LU NL SE**

(71) Anmelder: **F.Hoffmann-La Roche & Co. Aktiengesellschaft, Abt. VIII-Pt, CH-4002 Basel (CH)**

(72) Erfinder: **Reiner, Roland, Dr., Rheinfelderstrasse 8, CH-4058 Basel (CH)**

(74) Vertreter: **Mezger, Wolfgang, Dr. et al, Grenzacherstrasse 124 Postfach 601, CH-4002 Basel (CH)**

(54) **Chemotherapeutische Präparate und deren Herstellung.**

(57) Chemotherapeutische Präparate mit einem Gehalt an Fluoramphenicol und deren Herstellung in an sich bekannter Weise.

EP 0 007 995 A1

F. Hoffmann-La Roche & Co. Aktiengesellschaft, Basel, Schweiz

RAN 4410/130

### Chemotherapeutische Präparate und deren Herstellung

Fluoramphenicol (D-threo-1-(p-Nitrophenyl)-2-difluor-acetamino-1,3-propandiol) ist eine bekannte Substanz, die in der Literatur als biologisch unwirksam beschrieben worden ist (J. Pharm. Pharmacol. $\underline{3}$ [1951], 143) und auch bis heute keinen Eingang in die Chemotherapie gefunden hat.

Es wurde nun gefunden, dass Fluoramphenicol entgegen den früheren Befunden ein untoxisches, hochwirksames Breitspektrum-Antibioticum darstellt, das gegenüber dem bekannten Chloramphenicol in toxikologischer, chemotherapeutischer, pharmakokinetischer und/oder pharmazeutisch-anwendungstechnischer Hinsicht Vorteile bietet, wie den nachstehenden Daten entnommen werden kann:

1a) Akute Toxizität von Fluoramphenicol (Maus): $LD_{50}$ = 3000-4000 mg/kg p.o., 2000-3000 mg/kg i.p.

Mez/15.6.79

1b)  Akute Toxizität von Chloramphenicol (Maus): $LD_{50}$ = 2000-3000 mg/kg p.o., 1000-1500 mg/kg i.p.

2)   Cytotoxizität gegenüber menschlichen Lymphozyten: Fluoramphenicol ist rund 3mal weniger toxisch als Chloramphenicol.

3)   Cytotoxizität gegenüber KB-Zellen: Fluoramphenicol ist 3 bis 5mal weniger toxisch als Chloramphenicol.

4)   In-vivo-Aktivität von Fluoramphenicol (Maus) ($CD_{50}$ p.o.): 11 mg/kg gegen Streptococcus pyogenes ß15 [Chloramphenicol: 22 mg/kg], 33 mg/kg gegen Pseudomonas aeruginosa BA [Chloramphenicol: 93 mg/kg], 6,6 mg/kg gegen E. coli 1346 [Chloramphenicol: 6,6 mg/kg], 19 mg/kg gegen Salmonella typhimurium [Chloramphenicol: 18 mg/kg], 5,2 mg/kg gegen Proteus mirabilis [Chloramphenicol: 5,8 mg/kg].

5)   Fluoramphenicol gibt nach oraler Verabreichung (an der Ratte) höhere Serumkonzentrationen als Chloramphenicol und wird 2 bis 3mal langsamer ausgeschieden. Fluoramphenicol erzeugt ferner rund 10mal höhere Urinkonzentrationen (in den ersten 4 Stunden nach der Verabreichung) als Chloramphenicol. Die totale Urin-recovery an aktivem Fluoramphenicol ist zudem ebenfalls ca. 10mal höher als diejenige an aktivem Chloramphenicol.
Daraus wird geschlossen, dass Fluoramphenicol, im Gegensatz zu Chloramphenicol, metabolisch beständig ist, was insofern von Bedeutung ist, als die beim Chloramphenicol beobachteten irreversiblen hämatologischen Nebenwirkungen (aplastische Anämie) durch einen Metaboliten ausgelöst werden.

6)   Fluoramphenicol zeichnet sich durch unerwartet hohe Wasserlöslichkeit aus und eignet sich demgemäss, wiederum im Gegensatz zu Chloramphenicol, gut zur Herstellung wässriger galenischer Formen.

Die Herstellung der chemotherapeutischen Präparate kann in an sich bekannter Weise erfolgen, dadurch, dass man Fluoramphenicol mit zur therapeutischen Verabreichung geeigneten, an sich in solchen Präparaten üblichen Ingredienzien vermischt und/oder in eine geeignete Applikationsform bringt.

Wie gefunden wurde, lassen sich mit Fluoramphenicol wässrige Lösungen in Konzentrationen bis zu 0,5 g/10 ml herstellen. Solche Lösungen stellen eine wertvolle parenterale Applikationsform, insbesondere für die intravenöse Injektion und -Infusion dar. Dank seiner guten Wasserlöslichkeit eignet sich Fluoramphenicol auch für die Herstellung von ophthalmologischen Präparaten mit besonders guter Verträglichkeit, insbesondere für die Herstellung von Augentropfen.

Als wichtige Indikationen für Fluoramphenicol können Typhus, Paratyphus, Meningitis, Peritonitis, schwere Luftwegs- und Harnwegsinfektionen angegeben werden. Für Erwachsene kommt bei oraler oder parenteraler Anwendung eine Dosierung von 1,5 g pro Tag in 3 Einzelgaben in Betracht.

Für die Herstellung der erfindungsgemässen chemotherapeutischen Präparate kommen die bekannten, insbesondere die von der Galenik des Chloramphenicols her bekannten Materialien und Methoden in Betracht, wobei natürlich die bessere Wasserlöslichkeit des Fluoramphenicols einen breiteren Spielraum lässt.

Als Präparate kommen feste, halbfeste und flüssige Arzneiformen für die orale, perorale und parenterale Applikation in Frage, wie Tabletten, Kapseln, Dragées, Suppositorien, Pflaster, Salben, Crèmes, Gele, Lyophilisate, Sprays, Lösungen, Suspensionen oder Emulsionen, die den Wirkstoff in der Regel in Mischung mit einem geeigneten inerten Trägermaterial bei allfälliger gleichzeitiger Anwesenheit weiterer üblicher Hilfsstoffe, wie Konservierungs-, Stabilisierungs-, Netz- oder Emulgiermittel, Puffersubstanzen, Mittel zur geschmacklichen Verbesserung oder zur Veränderung des osmotischen Druckes, enthalten.

## Beispiel 1

Es wird in üblicher Weise eine Gelatine-Steckkapsel folgenden Inhaltes hergestellt:

| | |
|---|---|
| Fluoramphenicol | 500 mg |
| Luviskol | 20 mg |
| Mannit | 20 mg |
| Talk | 15 mg |
| Magnesiumstearat | 2 mg |

## Beispiel 2

Es wird in üblicher Weise ein Lyophilisat folgender Zusammensetzung hergestellt:

| | | |
|---|---|---|
| Fluoramphenicol | 500 | mg |
| p-Hydroxybenzoesäure-methylester | 0,5 | mg |
| p-Hydroxybenzoesäure-propylester | 0,05 | mg |

Dieses Lyophilisat wird in eine Ampulle abgefüllt. Eine derartige Ampulle enthält die lyophilisierten Wirkstoffe für 20 ml spritzfertiger Lösung. Zur Herstellung von 20 ml spritzfertiger Lösung werden dem Lyophilisat 19,5 ml destilliertes Wasser zugegeben.

## Beispiel 3

Es wird in üblicher Weise eine 1%ige sterile wässrige Lösung von Fluoramphenicol hergestellt, die zur Applikation von Augentropfen verwendet werden kann.

0007995

## Patentansprüche

1. Chemotherapeutische Präparate, gekennzeichnet durch einen Gehalt an Fluoramphenicol.

2. Sterile wässrige Lösungen, gekennzeichnet durch einen Gehalt an Fluoramphenicol.

3. Wässrige Injektions- oder Infusionslösungen, gekennzeichnet durch einen Gehalt an Fluoramphenicol.

4. Verfahren zur Herstellung chemotherapeutischer Präparate, dadurch gekennzeichnet, dass man Fluoramphenicol mit zur therapeutischen Verabreichung geeigneten, an sich in solchen Präparaten üblichen Ingredienzien vermischt und/oder in eine geeignete Applikationsform bringt.

***

Europäisches
Patentamt

**EUROPÄISCHER RECHERCHENBERICHT**

000**7995**

Nummer der Anmeldung

EP 79 10 2213

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | CHEMICAL ABSTRACTS, Band 45, Nr. 18, veröffentlicht am 25. September 1951, Seite 7978f – Seite 7979c Columbus, Ohio, U.S.A. B.N. FEITELSON et al.: "Structural requirements in the amphenicol series for antibiotic activity" & J. PHARM. PHARMACOL. 3, 149-59 (1951) * Zusammenfassung * | 1 |
| A | US - A - 2 483 885 (HARRY M. CROOKS) * Spalte 1, Zeilen 16-23; Spalten 7,8; Beispiel 10 * | 1 |

**KLASSIFIKATION DER ANMELDUNG (Int.Cl. 3)**

A 61 K 31/16

**RECHERCHIERTE SACHGEBIETE (Int. Cl. 3)**

A 61 K 31/16
C 07 C 103/38

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 13-11-1979 | BRINKMANN |

EPA form 1503.1 06.78